# EUROPEAN PATENT APPLICATION

(11) **EP 4 173 486 A1**
(43) Date of publication of application: **03.05.2023**
(21) Application number: 21829262.1
(22) Date of filing: 03.03.2021
(51) Int. Cl.: A01P 1/00, A61L 9/01, A01N 25/02, A01N 59/00, A01N 59/08

(54) **COMPOSITION FOR INHIBITING BINDING OF SARS-COV-2 TO ACE2 PROTEIN**

(30) Priority: 24.06.2020 JP 2020108856
(71) Applicant: Taiko Pharmaceutical Co., Ltd., Suita-shi Osaka 564-0032 (JP)
(72) Inventor: OGATA, Norio, Suita-shi, Osaka 564-0032 (JP); MIURA, Takanori, Suita-shi, Osaka 564-0032 (JP)
(74) Representative: Heubeck, Christian
(86) International application number: PCT/JP2021/008093
(87) International publication number: WO 2021/261020

(57) **Abstract**

The present invention searches for compounds etc. that may inhibit the interaction between the spike (S) protein of SARS-CoV-2 and the ACE2 protein. The present invention provides a composition for inhibiting the binding between the spike (S) protein of SARS-CoV-2 and the angiotensin converting enzyme 2 (ACE2) protein comprising an effective amount of chlorine dioxide.

## Description

### Technical Field

The present invention relates to a composition for inhibiting the binding between SARS-CoV-2 and ACE2 protein.

### Background Art

SARS-CoV-2 (or 2019-nCoV) is a virus belonging to the family Coronaviridae having single-stranded plus strand RNA viral genome. From 2019 to 2020, infection due to SARS-CoV-2 (COVID-19) was epidemic throughout the world, and numerous individuals became infected. SARS-CoV-2 is known to infect animals by utilizing the interaction between the spike (S) protein which is one of viral proteins and the angiotensin converting enzyme 2 (ACE2) protein which is present on the animal cell surface. However, at this stage, no agents etc. that effectively inhibit the interaction have been found.

While chlorine dioxide gas is a gas safe for the living animal body at low concentrations, it is known to have inactivation or deodorization actions etc. against microorganisms such as bacteria, fungus, and virus even at such concentrations (Patent Literature 1). However, the effect of chlorine dioxide against SARS-CoV-2 has not yet been verified.

### Citation List

[Patent Literature 1] WO2007/061092

### Summary of the Invention

### Problems to be Solved by the Invention

The object of the present invention is to search for compounds etc. that may inhibit the interaction between the spike (S) protein of SARS-CoV-2 and the ACE2 protein.

### Means for Solving the Problems

As a result of the present inventors searching for compounds etc. that may inhibit the interaction between the spike protein of SARS-CoV-2 and the ACE2 protein, it was surprisingly found that chlorine dioxide acts on the spike protein of SARS-CoV-2 and inhibits the interaction between the spike protein and the ACE2 protein.

In other words, in one embodiment, the present invention relates to a composition comprising an effective amount of chlorine dioxide for inhibiting the binding between the spike protein of SARS-CoV-2 and the ACE2 protein.

In one embodiment of the present invention, it is characterized that the composition is applied to a site at which SARS-CoV-2 may exist.

In one embodiment of the present invention, it is characterized that the ACE2 protein is a human ACE2 protein.

In one embodiment of the present invention, it is characterized that the composition is a liquid comprising chlorine dioxide.

In one embodiment of the present invention, it is characterized that the concentration of chlorine dioxide in the liquid is 1 - 2000 ppm.

In one embodiment of the present invention, it is characterized that the liquid comprises 10 - 2000 ppm of chlorine dioxide.

In one embodiment of the present invention, it is characterized that the liquid comprises 10 - 1000 ppm of chlorine dioxide.

In one embodiment of the present invention, it is characterized that the liquid further comprises a chlorite.

In one embodiment of the present invention, it is characterized that the liquid comprises chlorine dioxide that is prepared separately from the chlorite.

In one embodiment of the present invention, it is characterized that the chlorine dioxide in the liquid is entirely derived from the chlorite.

In one embodiment of the present invention, it is characterized that the concentration of the chlorite in the liquid is 0.05 wt% - 10.0 wt%.

In one embodiment of the present invention, it is characterized that the concentration of the chlorite in the liquid is 0.1 wt% - 5.0 wt%.

In one embodiment of the present invention, it is characterized that the pH of the liquid is adjusted to be within the range of 4.5 - 6.5.

In one embodiment of the present invention, it is characterized that the pH of the liquid is adjusted to be within the range of 5.5 - 6.0.

In one embodiment of the present invention, it is characterized that the liquid further comprises a gelling agent.

In one embodiment of the present invention, it is characterized that the liquid is in a gel state.

In one embodiment of the present invention, it is characterized that the composition is a gaseous composition comprising gaseous chlorine dioxide.

In one embodiment of the present invention, it is characterized that the composition is applied so that the chlorine dioxide gas concentration within the space will be 0.00001 ppm - 0.3 ppm as the concentration that inhibits the binding between the spike protein of SARS-CoV-2 and the ACE2 protein.

In one embodiment of the present invention, it is characterized that the "site at which SARS-CoV-2 may exist" is a site where a human hand comes in contact.

In one embodiment of the present invention, it is characterized that the "site at which SARS-CoV-2 may exist" is a space where a human being breathes.

In one embodiment of the present invention, it is characterized that the composition inhibits the binding between the spike protein of SARS-CoV-2 and the ACE2 protein by 30% or more compared to when chlorine dioxide is absent.

In one embodiment of the present invention, it is characterized that the composition shows 30% or more inhibition of interaction compared to when chlorine dioxide is absent in an inhibition test for the interaction between the spike protein of SARS-CoV-2 and the ACE2 protein employing "SARS-CoV-2 Spike:ACE2 Inhibitor Screening Assay Kit (from BPS Bioscience, Product Number #79931)."

Other embodiments of the present invention relate to a method for inhibiting the binding between the spike protein of SARS-CoV-2 and the ACE2 protein, comprising a step of applying an effective amount of chlorine dioxide to a site at which SARS-CoV-2 may exist.

Other embodiments of the present invention relate to a method for inhibiting the binding between the spike (S) protein of SARS-CoV-2 and the angiotensin converting enzyme 2 (ACE2) protein, comprising a step of applying a gaseous composition comprising an effective amount of chlorine dioxide to a site at which SARS-CoV-2 may exist.

In one embodiment of the present invention, it is characterized that the step of applying a gaseous composition comprising an effective amount of chlorine dioxide to a site at which SARS-CoV-2 may exist is a step of applying the gaseous composition comprising chlorine dioxide so that the chlorine dioxide gas concentration at the site will be 0.00001 ppm - 0.3 ppm.

In one embodiment of the present invention, it is characterized that the step of applying a gaseous composition comprising an effective amount of chlorine dioxide to a site at which SARS-CoV-2 may exist is a step of applying the gaseous composition comprising chlorine dioxide so that the chlorine dioxide gas concentration at the site will be 0.3 ppm - 230 ppm.

In one embodiment of the present invention, it is characterized that the step of applying a gaseous composition comprising an effective amount of chlorine dioxide to a site at which SARS-CoV-2 may exist is a step of applying the gaseous composition comprising chlorine dioxide so that the chlorine dioxide gas concentration at the site will be 10 ppm - 230 ppm.

Note that an invention of any combination of the above one or more characteristics of the present invention is also encompassed by the scope of the present invention.

### Description of Embodiments

The present invention utilizes a novel knowledge that chlorine dioxide inhibits the binding between the spike S protein of SARS-CoV-2 and the ACE2 protein.

According to the present invention, by applying chlorine dioxide to a site at which SARS-CoV-2 may exist, the binding activity of the spike protein of SARS-CoV-2 towards the ACE2 protein is inactivated, and the development of infection due to SARS-CoV-2 (COVID-19) can be prevented.

When referring to "SARS-CoV-2" in the present disclosure, the term refers to a virus that is classified as "SARS-CoV-2" from a taxonomical standpoint, and such a classification is made by e.g. the International Committee on Taxonomy of Viruses. Typically, SARS-CoV-2 is a coronavirus that infects animals by utilizing the binding between the spike protein and the ACE2 protein.

Note that in a modified example of the present invention, the subject thereof is not limited to SARS-CoV-2, but may be a coronavirus that infects animals by utilizing the binding between the spike protein and the ACE2 protein.

The use of chlorine dioxide in the present invention may be the use of a liquid comprising chlorine dioxide (chlorine dioxide liquid) or the use of a gaseous composition comprising gaseous chlorine dioxide.

As one embodiment of the present invention, when employing a liquid comprising chlorine dioxide, e.g. by applying the liquid to a site where a human hand comes in contact, the binding activity of the spike protein of SARS-CoV-2 that may exist at the site towards the ACE2 protein is inactivated, and the development of infection due to SARS-CoV-2 (COVID-19) can be prevented.

Non-limiting examples of the "site where a human hand comes in contact" include equipment in living space, a commercial facility, a public facility, a medical facility, transportation, and the like. Non-limiting examples of equipment can include floor, table, toilet, kitchen, bathroom, washroom, entrance hall, trash can, furniture, doorknob, handrail, vehicle steering wheel, switch for electronic instruments etc., stationery products such as writing utensils, tableware, and the like. Particularly in a medical facility, by applying the present invention on an object or site that came in contact with a patient infected with or is suspected of being infected with SARS-CoV-2, the risk of nosocomial infection can be reduced.

Moreover, as one embodiment of the present invention, when employing a gaseous composition comprising gaseous chlorine dioxide, e.g. by applying an effective amount or the gaseous composition to a space where a human being breathes, the binding activity of the spike protein of SARS-CoV-2 that may exist at the space towards the ACE2 protein is inactivated, and the development of infection due to SARS-CoV-2 (COVID-19) can be prevented.

The "space where a human being breathes" may be a closed space or an open space, although the effect of the present invention is more easily obtained in a space that is close to a closed space since the gaseous composition of the present invention is less easily diffused. Non-limiting examples of the "space where a human being breathes" include space in living space, a commercial facility, a public facility, a medical facility, transportation, and the like. Particularly in a medical facility, by using the present invention in a space where a patient infected with or is suspected of being infected with SARS-CoV-2 is present, the risk of nosocomial infection can be reduced.

The method for preparing the chlorine dioxide liquid that can be employed in the present invention is not limited, and liquids prepared by various well-known methods can be employed. For example, since chlorine dioxide has high solubility in water, chlorine dioxide liquid can be prepared by dissolving gaseous chlorine dioxide in a solvent such as water. Moreover, since chlorite produces chlorine dioxide under acidic conditions, a pH-adjusted aqueous chlorite solution can also be used as the chlorine dioxide liquid (in this case, the chlorine dioxide in the liquid will all be derived from chlorite.) Moreover, a chlorine dioxide liquid can also be prepared by electrolyzing a given electrolytic solution comprising chlorite.

It is preferred that the chlorine dioxide liquid that can be employed in the present invention is a liquid having 1 - 2000 ppm of chlorine dioxide dissolved therein. An example of a more preferred liquid can include a liquid that has 1 - 2000 ppm of chlorine dioxide dissolved therein, comprises chlorite at a concentration of 0.05 wt% - 10 wt%, and has the pH of the liquid adjusted to be in the range of 4.5 - 6.5. The concentration of chlorine dioxide comprised in the chlorine dioxide liquid may be 10 - 2000 ppm, may be 10 - 1000 ppm, preferably may be 50 - 800 ppm, and further preferably may be 100 - 600 ppm. Moreover, the concentration of the chlorite comprised in the chlorine dioxide liquid may be 0.1 wt% - 5.0 wt%, and more preferably may be 0.5 wt% - 2.5 wt%.

In regards to the chlorine dioxide liquid comprising a chlorite, when the pH of the chlorine dioxide liquid becomes lower than 4.5, the chlorite in the liquid reacts excessively and chlorine dioxide gas becomes easily released, and thus, controlling of the amount of chlorine dioxide gas released becomes difficult, and the likelihood of reduced preservation stability of the chlorine dioxide liquid increases. Moreover, when the pH of the chlorine dioxide liquid becomes higher than 6.5, the reactivity of the chlorite in the liquid becomes easily reduced, and the likelihood that an appropriate amount of chlorine dioxide gas will not be released increases. It is more preferred that the pH of the chlorine dioxide liquid is within the range of 5.5 - 6.0. Note that the concentration of chlorine dioxide in the chlorine dioxide liquid, the chlorite concentration, and pH can be in any combination within the above range.

The chlorine dioxide liquid that can be employed in the present invention can be for example manufactured as follows. First, (a) a chlorite is dissolved in water to prepare an aqueous chlorite solution at 2000 - 180000 ppm, (b) an aqueous chlorine dioxide solution at 100 - 2900 ppm having chlorine dioxide gas dissolved therein is separately prepared, and after mixing (a) and (b), this solution is mixed with a pH adjuster to adjust the pH to 4.5 - 6.5. Note that the aqueous chlorite solution concentration and the aqueous chlorine dioxide solution concentration in the above manufacturing method can be appropriately adjusted by those skilled in the art according to the composition of the intended liquid.

By preparing the liquid according to the above method, the concentration of chlorine dioxide dissolved in the liquid can be freely adjusted from high concentrations to low concentrations. Moreover, since the liquid prepared with the above method comprises chlorine dioxide gas and chlorite, when chlorine dioxide gas is released from the liquid into the air, the chlorine dioxide gas concentration in the liquid is reduced, but chlorine dioxide is supplied into the liquid from the chlorite by chemical equilibrium, and as a result, the chlorine dioxide gas concentration in the liquid is retained virtually constant. Due to this effect, the above chlorine dioxide liquid is able to release chlorine dioxide gas into the air in a controlled-release manner over an extended period of time. Moreover, when the liquid is applied directly to the subject, since this can provide more chloride dioxide to the subject than a liquid with merely chlorine dioxide gas dissolved in water, a higher effect may be exerted. Because the balance between the amount of chlorine dioxide gas released from the liquid and the supply of chlorine dioxide from chlorite is favorably retained when the pH of the liquid is adjusted to be within the range of 4.5 - 6.5, chlorine dioxide gas can be released at a virtually constant concentration for a longer period of time.

Chlorites that may be comprised in the chlorine dioxide liquid that can be employed in the present invention include, e.g., alkali metal chlorites or alkaline earth metal chlorites. Alkali metal chlorites include, e.g., sodium chlorite, potassium chlorite, and lithium chlorite, and alkaline earth metal chlorites include calcium chlorite, magnesium chlorite, and barium chlorite. Among these, sodium chlorite and potassium chlorite are preferred in terms of being readily available, and sodium chlorite is most preferred. These chlorites may be used alone, or two or more may be used in combination.

Those skilled in the art can employ any pH adjuster for preparing the chlorine dioxide liquid that can be employed in the present invention. For example, phosphoric acid, boric acid, metaphosphoric acid, pyrophosphoric acid, sulfamic acid, acetic acid, citric acid, or a salt thereof etc. can be employed, and an inorganic acid or a salt thereof is preferred in that superior preservation stability is obtained. Among these, use of phosphoric acid or a salt thereof (such as a mixture of sodium dihydrogenphosphate, sodium dihydrogenphosphate, and disodium hydrogenphosphate) is preferred, and use of sodium dihydrogenphosphate is further preferred, in terms of being superior in preservation stability and being able to suppress the variation in liquidity (pH) during preservation to a minimum. Note that the pH adjuster may be used alone, or two or more may be used in combination.

The chlorine dioxide liquid that can be employed in the present invention may be made into a gel composition by further adding a gelling agent (in the present specification, such a gel composition is also called a "liquid.") By making the chlorine dioxide liquid into a gel composition, chlorine dioxide can be applied to the subject in a controlled-release manner for a longer period of time. Non-limiting examples of gelling agents can include high water-absorbing resins (such as starch-based water-absorbing resins, cellulose-based water-absorbing resins, and synthetic polymer-based water-absorbing resins.) Note that a gel composition comprising the chlorine dioxide liquid that can be employed in the present invention may be provided as a kit to be used by mixing the chlorine dioxide liquid and the gelling agent at the time of need.

When using a gaseous composition comprising chlorine dioxide gas in the present invention, it is preferred that the chlorine dioxide gas concentration within the space is set at a concentration that inhibits the binding between the spike protein of SARS-CoV-2 and the ACE2 protein (such as 0.00001 ppm or higher).

When using the gaseous composition comprising chlorine dioxide gas in a space wherein humans or animals are present, it is preferred that the target the chlorine dioxide gas concentration within the space is set at 0.00001 ppm - 0.3 ppm. The lower limit of the concentration of chlorine dioxide may be arbitrary selected from among e.g. 0.00001 ppm, 0.0001 ppm, 0.001 ppm, 0.01 ppm, and 0.1 ppm. The upper limit of the concentration of chlorine dioxide may be arbitrary selected from among e.g. 0.3 ppm, 0.2 ppm, and 0.1 ppm. Preferred range of the concentration of chlorine dioxide can include 0.0001 ppm - 0.3 ppm, 0.0001 ppm - 0.2 ppm, 0.0001 ppm - 0.1 ppm, 0.001 ppm - 0.3 ppm, 0.001 ppm - 0.2 ppm, 0.001 ppm - 0.1 ppm, 0.01 ppm - 0.3 ppm, 0.01 ppm - 0.2 ppm, 0.01 ppm - 0.1 ppm, and 0.1 ppm - 0.3 ppm.

The duration for supplying chlorine dioxide gas into space with the present invention is not particularly limited, and the duration for supplying may be appropriately adjusted according to the chlorine dioxide gas concentration to be supplied. For example, when the chlorine dioxide gas concentration in a space is set at 0.00001 ppm - 0.01 ppm, there is no problem in continuing a constant supply of chlorine dioxide gas. When the chlorine dioxide gas concentration in a space is set at 0.01 ppm - 0.1 ppm, it is preferred to set the duration for supplying chlorine dioxide gas into space at 10 minutes - 480 minutes, more preferably 15 minutes - 90 minutes, and further preferably 15 minutes - 60 minutes. Moreover, when the chlorine dioxide gas concentration in a space is set at 0.1 ppm - 0.3 ppm, it is preferred that the duration for supplying chlorine dioxide gas into space is set at 0.5 minutes - 480 minutes, more preferably 1 minute - 60 minutes, and further preferably 2 minutes - 15 minutes.

When employing the gaseous composition comprising chlorine dioxide gas for decontamination of SARS-CoV-2 (so-called "fumigation") in a space wherein humans or animals are not present, the target the chlorine dioxide gas concentration within the space may be 0.3 ppm or higher. The lower limit of the concentration of chlorine dioxide may be arbitrary selected from among e.g. 0.3 ppm, 0.5 ppm, 1.0 ppm, 2.0 ppm, 3.0 ppm, 4.0 ppm, and 5.0 ppm. With respect to ensuring inhibition of the binding between the spike protein of SARS-CoV-2 and the ACE2 protein, the chlorine dioxide gas concentration may be 10 ppm or higher, 15 ppm or higher, or 20 ppm or higher. Note that with respect to preventing corrosion of equipment etc. within the space, the chlorine dioxide gas concentration may be set at 230 ppm or lower, 200 ppm or lower, 150 ppm or lower, 100 ppm or lower, 75 ppm or lower, or 50 ppm or lower. Preferred range of the concentration of chlorine dioxide can include 0.3 ppm - 230 ppm, 0.5 ppm - 230 ppm, 1.0 ppm - 230 ppm, 2.0 ppm - 230 ppm, 3.0 ppm - 230 ppm, 4.0 ppm - 230 ppm, 5.0 ppm - 230 ppm, 10.0 ppm - 230 ppm, 15.0 ppm - 230 ppm, 20.0 ppm - 230 ppm, 0.3 ppm - 200 ppm, 0.5 ppm - 200 ppm, 1.0 ppm - 200 ppm, 2.0 ppm - 200 ppm, 3.0 ppm - 200 ppm, 4.0 ppm - 200 ppm, 5.0 ppm - 200 ppm, 10.0 ppm - 200 ppm, 15.0 ppm - 200 ppm, 20.0 ppm - 200 ppm, 0.3 ppm - 150 ppm, 0.5 ppm - 150 ppm, 1.0 ppm - 150 ppm, 2.0 ppm - 150 ppm, 3.0 ppm - 150 ppm, 4.0 ppm - 150 ppm, 5.0 ppm - 150 ppm, 10.0 ppm - 150 ppm, 15.0 ppm - 150 ppm, 20.0 ppm - 150 ppm, 0.3 ppm - 100 ppm, 0.5 ppm - 100 ppm, 1.0 ppm - 100 ppm, 2.0 ppm - 100 ppm, 3.0 ppm - 100 ppm, 4.0 ppm - 100 ppm, 5.0 ppm - 100 ppm, 10.0 ppm - 100 ppm, 15.0 ppm - 100 ppm, 20.0 ppm - 100 ppm, 0.3 ppm - 75 ppm, 0.5 ppm - 75 ppm, 1.0 ppm - 75 ppm, 2.0 ppm - 75 ppm, 3.0 ppm - 75 ppm, 4.0 ppm - 75 ppm, 5.0 ppm - 75 ppm, 10.0 ppm - 75 ppm, 15.0 ppm - 75 ppm, 20.0 ppm - 75 ppm, 0.3 ppm - 50 ppm, 0.5 ppm - 50 ppm, 1.0 ppm - 50 ppm, 2.0 ppm - 50 ppm, 3.0 ppm - 50 ppm, 4.0 ppm - 50 ppm, 5.0 ppm - 50 ppm, 10.0 ppm - 50 ppm, 15.0 ppm - 50 ppm, and 20.0 ppm - 50 ppm.

When using a gaseous composition comprising chlorine dioxide gas in the present invention, the supply source of the chlorine dioxide gas is not limited, and various methods and devices can be used. For example, well-known chlorine dioxide generators, chlorine dioxide generating agents, kits for generating chlorine dioxide, and the like can be employed as the supply source of the chlorine dioxide gas. Moreover, the aforementioned chlorine dioxide liquids or gel compositions may also be employed as the supply source of the chlorine dioxide gas.

The terms used herein are employed for describing particular embodiments, and do not intend to limit the invention.

Moreover, the term "comprising" as used herein, unless the content clearly indicates to be understood otherwise, intends the presence of the described items (such as components, steps, elements, or numbers), does not exclude the presence of other items (such as components, steps, elements, and numbers.)

Unless otherwise defined, all terms used herein (including technical and scientific terms) have the same meanings as those broadly recognized by those skilled in the art of the technology to which the present invention belongs. The terms used herein, unless explicitly defined otherwise, should be construed as having meanings consistent with the meanings herein and in related technical fields, shall not be construed as having idealized or excessively formal meanings.

The embodiments of the present invention may be described with reference to schematic diagrams. In such a case, they may be exaggerated in presentation in order to allow clear description.

In the present specification, for example, when expressed as "1 - 10%, " those skilled in the art will recognize that the expression refers individually and specifically to 1, 2, 3, 4, 5, 6, 7, 8, 9, or 10%.

In the present specification, any and all numeric values employed for indicating component contents or numeric value ranges is, unless explicitly indicated, construed as encompassing the meaning of the term "about." For example, "10-folds," unless explicitly indicated, is recognized as meaning "about 10-folds."

All of the disclosures of the literatures cited herein should be deemed as cited herein, and those skilled in the art will cite and recognize the related disclosed contents in these prior art literatures as a part of the present specification according to the context herein without departing from the spirit and scope of the present invention.

The present invention will now be described in further detail with reference to Examples. However, the present invention can be embodied by various aspects, shall not be construed as being limited to the Examples described herein.

### Examples

### [Example 1: Inhibition of Binding Between Spike Protein of SARS-CoV-2 and ACE2 Protein by Chlorine Dioxide Liquid]

### Materials and Methods

Chlorine dioxide gas generated by adding hydrochloric acid to aqueous sodium chlorite solution was introduced into distilled water to prepare as a 55 mM aqueous chlorine dioxide solution. This was shaded from light until immediately before use, and stored at 4°C in an air-tight container.

Regarding the inhibition experiment of the binding between SARS-CoV-2 and ACE2 protein, SARS-CoV-2 Spike:ACE2 Inhibitor Screening Assay Kit (Product Number #79931) from BPS Bioscience (San Diego, California) was employed. This kit comprises purified spike (S) protein of SARS-CoV-2 and ACE2 protein. The experiment was carried out following the protocol of the manufacturer.

First, the spike protein was treated at room temperature for 5 minutes with 0, 0.25, and 0.5 mM aqueous chlorine dioxide solutions, and then 3.5 µg/ml of the ACE2 protein was added to the reaction solution and left still at 25°C for 30 minutes. To the reaction solution was added twice the molar amount of sodium thiosulfate to quench the reaction. Free ACE2 protein was then removed, and the amount of ACE2 protein bound to the spike protein was measured. Peroxidase-labeled anti-ACE2 antibody was employed for this measurement. After adding this antibody to the reaction system, peroxidase was utilized to measure the amount of antibody bound to the ACE2 protein. In other words, after adding the peroxidase substrate, the strength of the chemiluminescence produced was measured with a luminometer.

### Results

Experimental results are shown in the following table. [Table 1]

**Table 1: Experimental Results**

| Concentration of Chlorine Dioxide | Luminescence Intensity (counts/sec x 1000, average value ± standard deviation, n = 4) |
|---|---|
| 0 mM | 6097 ± 708 |
| 0.25 mM | 3107 ± 709 |
| 0.5 mM | 116 ± 17 |

As shown in Table 1, the binding between the spike protein of SARS-CoV-2 and the ACE2 protein was inhibited chlorine dioxide concentration-dependently. Note that the binding between the spike protein and the ACE2 protein was significantly reduced (p < 0.01; t-test against 0 mM) even for the lowest concentration of chlorine dioxide tested here, 0.25 mM (about 17 ppm (w/w).)

From the above results, it was shown that chlorine dioxide has the effect of inhibiting the binding between the spike protein of SARS-CoV-2 and the ACE2 protein.

### [Example 2: Inhibition of Binding Between Spike Protein of SARS-CoV-2 and ACE2 Protein by Chlorine Dioxide Gas]

### Materials and Methods

Regarding the inhibition experiment of the binding between the spike protein of SARS-CoV-2 and the ACE2 protein, SARS-CoV-2 Spike:ACE2 Inhibitor Screening Assay Kit (Product Number #79931) from BPS Bioscience (San Diego, California) was employed.

First, the spike protein of SARS-CoV-2 S1 protein was adsorbed onto the surface of each well of a 96-well microtiter plate, and subsequently 50 microliters of buffer (Immuno Buffer 1 of the kit) was placed in each well of the plate. Next, the wells were exposed various concentrations of chlorine dioxide gas at 25°C for 6 minutes. The exposure was carried out in a box of 24.5 x 17.5 x 17.0 cm (volume 7.29 L) having an inner surface made from aluminum. For chlorine dioxide, a chlorine dioxide generator (Cleverin Gel 150 g (Taiko Pharmaceutical Co.,Ltd.)) was placed in the box beforehand, and the chlorine dioxide gas concentration in the box immediately before placing the plate was measured. Note that the wells not exposed to chlorine dioxide gas were covered with vinyl tape. After exposure, 10 microliters of 10 mmol/L aqueous sodium thiosulfate solution was added to each well to stop the influence of chlorine dioxide. Immediately after this, aqueous ACE2 protein solution was added, and the binding ability of S1 protein against ACE2 was measured according to the kit protocol in liquid phase. Measurement was made with chemiluminescence quantification employing SH-9000 from CORONA. Each chlorine dioxide gas concentration was measured at 3 points (n = 3,) and the results were determined as average values and standard deviations thereof. Statistical significance test of each data was performed with t-test.

### Results

Experimental results are shown in the following table.

### [Table 2]

**Table 2: Experimental Results**

| Chlorine Dioxide Gas Concentration (ppm, vol/vol) | Luminescence Intensity (counts/sec x 1000, average ± standard deviation, n = 3) | p value against 0 ppm |
|---|---|---|
| 0 | 1630 ± 59 | - |
| 3.3 | 1322 ± 45 | 0.0018 |
| 22 | 1072 ± 47 | 0.00021 |

As shown in Table 2, the binding between the spike protein of SARS-CoV-2 and the ACE2 protein was inhibited chlorine dioxide gas concentration-dependently.

From the above results, it was shown that chlorine dioxide gas has the effect of inhibiting the binding between the spike protein of SARS-CoV-2 and the ACE2 protein.

## Claims

1. A composition for inhibiting the binding between the spike (S) protein of SARS-CoV-2 and the angiotensin converting enzyme 2 (ACE2) protein, wherein the composition is a liquid comprising chlorine dioxide at a concentration of 10 - 2000 ppm.

2. The composition according to claim 1, **characterized in that** the composition is applied to a site at which SARS-CoV-2 may exist.

3. The composition according to claim 1, **characterized in that** the ACE2 protein is a human ACE2 protein.

4. The composition according to any one of claims 1 to 3, **characterized in that** the liquid further comprises a chlorite.

5. The composition according to claim 4, **characterized in that** the liquid comprises chlorine dioxide that is prepared separately from the chlorite.

6. The composition according to claim 4, **characterized in that** the chlorine dioxide in the liquid is entirely derived from the chlorite.

7. The composition according to claim 4, **characterized in that** the concentration of the chlorite in the liquid is 0.05 wt% - 10.0 wt%.

8. The composition according to claim 7, **characterized in that** the concentration of the chlorite in the liquid is 0.1 wt% - 5.0 wt%.

9. The composition according to any one of claims 1 to 8, **characterized in that** the pH of the liquid is adjusted to be within the range of 4.5 - 6.5.

10. The composition according to claim 9, **characterized in that** the pH of the liquid is adjusted to be within the range of 5.5 - 6.0.

11. The composition according to any one of claims 1 to 10, **characterized in that** the liquid further comprises a gelling agent.

12. The composition according to claim 11, **characterized in that** the liquid is in a gel state.

13. The composition according to claim 2, **characterized in that** the "site at which SARS-CoV-2 may exist" is a site where a human hand comes in contact.

14. The composition according to claim 2, **characterized in that** the "site at which SARS-CoV-2 may exist" is a space where a human being breathes.

15. The composition according to any one of claims 1 to 14, **characterized in that** the composition inhibits the binding between the spike protein of SARS-CoV-2 and the ACE2 protein by 30% or more compared to when chlorine dioxide is absent.

16. A method for inhibiting the binding between the spike protein of SARS-CoV-2 and the ACE2 protein, comprising a step of applying a liquid comprising chlorine dioxide at a concentration of 10 - 2000 ppm to a site at which SARS-CoV-2 may exist.

17. A method for inhibiting the binding between the spike (S) protein of SARS-CoV-2 and the angiotensin converting enzyme 2 (ACE2) protein, comprising a step of applying a gaseous composition comprising an effective amount of chlorine dioxide to a site at which SARS-CoV-2 may exist.

18. The method according to claim 17, wherein the step of applying a gaseous composition comprising an effective amount of chlorine dioxide to a site at which SARS-CoV-2 may exist is a step of applying the gaseous composition comprising chlorine dioxide so that the chlorine dioxide gas concentration at the site will be 0.00001 ppm - 0.3 ppm.

19. The method according to claim 17, wherein the step of applying a gaseous composition comprising an effective amount of chlorine dioxide to a site at which SARS-CoV-2 may exist is a step of applying the gaseous composition comprising chlorine dioxide so that the chlorine dioxide gas concentration at the site will be 0.3 ppm - 230 ppm.

20. The method according to claim 19, wherein the step of applying a gaseous composition comprising an effective amount of chlorine dioxide to a site at which SARS-CoV-2 may exist is a step of applying the gaseous composition comprising chlorine dioxide so that the chlorine dioxide gas concentration at the site will be 10 ppm - 230 ppm.
